# EUROPÄISCHE PATENTSCHRIFT

(11) **EP 1 126 790 B1**
(45) Veröffentlichungstag und Bekanntmachung des Hinweises auf die Patenterteilung: **10.08.2005**
(21) Anmeldenummer: 99957998.0
(22) Anmeldetag: 05.11.1999
(51) Int. Cl.: A61B 17/32, A61B 18/00

(54) **MEDIZINISCHES INSTRUMENT ZUM SCHNEIDEN VON GEWEBE IM MENSCHLICHEN ODER TIERISCHEN KÖRPER**
A MEDICAL INSTRUMENT FOR CUTTING TISSUE IN THE HUMAN OR ANIMAL BODY
INSTRUMENT MEDICAL POUR LE SECTIONNEMENT DE TISSU DANS LE CORPS D'UN ETRE HUMAIN OU D'UN ANIMAL

(30) Priorität: 05.11.1998 DE 19851006; 02.02.1999 DE 19904055; 22.07.1999 DE 19934536; 30.10.1999 DE 19953141
(43) Veröffentlichungstag der Anmeldung: 29.08.2001
(73) Patentinhaber: Scarfi, Andrea, Dr. med., 73760 Ostfildern (DE)
(72) Erfinder: Scarfi, Andrea, Dr. med., 73760 Ostfildern (DE)
(74) Vertreter: Heuckeroth, Volker, Dr.
(86) Internationale Anmeldenummer: PCT/EP1999/008483
(87) Internationale Veröffentlichungsnummer: WO 2000/027294

(56) Entgegenhaltungen:
- CH-A- 186 088
- DE-A- 19 544 523
- US-A- 2 127 190
- US-A- 4 452 106
- US-A- 5 509 923

## Beschreibung

Die Erfindung betrifft ein medizinisches Instrument zum Schneiden und bipolaren Koagulieren von Gewebe im menschlichen oder tierischen Körper, mit zwei um eine ortsfeste Drehachse relativ zueinander verschwenkbaren Scherenarmen, die jeweils eine Schneidkante aufweisen, wobei die Schneidkanten beim Verschwenken der Scherenarme über Kreuz streifend aneinander vorbeilaufen, wobei weiterhin die Drehachse an den Scherenarmen angeordnet ist, und wobei ferner die Scherenarme als mit Hochfrequenzstrom beaufschlagbare Elektroden ausgebildet sind, wobei sich gegenseitig berührende Bereiche des einen Scherenarms und des anderen Scherenarms elektrisch isoliert sind.

Ein solches Instrument ist aus DE-A-197-00 605 bekannt.

Ein derartiges Instrument dient in der minimal-invasiven Chirurgie zum Schneiden von Gewebe im menschlichen oder tierischen Körper.

Das aus der DE 197 00 605 A1 bekannte Instrument weist nach entgegengesetzten Seiten aufgespreizte gegeneinander verschwenkbare Scherenarme auf, die jeweils eine Schneidkante aufweisen, wobei die Schneidkanten beim Verschwenken der Scherenarme über Kreuz streifend aneinander vorbeilaufen. Mit dieser Ausgestaltung der Scherenarme soll Gewebe mechanisch geschnitten werden können.

An ihrem proximalen Ende weisen die Scherenarme jeweils einen Biegeabschnitt auf, auf den ein Schieberohr durch Betätigen einer Handhabe am proximalen Ende des Instruments aufschiebbar ist, wodurch die Scherenarme aus ihrer Spreizstellung zum Schneiden von Gewebe radial gegeneinander zusammengedrückt werden. Bei zurückgeschobenem Schieberohr spreizen die Scherenarme elastisch federnd auseinander. Ein solcher Betätigungsmechanismus für eine medizinische Schere ist auch aus der US 5,334,198 bekannt.

Bei einem ersten Ausführungsbeispiel des zuerst genannten bekannten Instruments sind die Scherenarme gelenklos ausgebildet, d.h. die Scherenarme sind nicht miteinander verbunden und sind auch sonst nicht beim Verschwenken geführt.

Dadurch werden die Scherenarme beim Schneiden von Gewebe, insbesondere von härterem Gewebe, wie bspw. von Gefäßen, in Richtung quer zur Längsrichtung der Scherenarme aufgespreizt bzw. verbogen und können dann ihre scherende Wirkung, die auf dem streifenden Vorbeilaufen der beiden Schneidkanten aneinander beruht, nicht mehr ausreichend entfalten, weil die Schneidkanten dann berührungslos aneinander vorbeilaufen können, mit dem Nachteil, daß die Schneidwirkung des Instruments unzureichend ist.

Bei einem weiteren Ausführungsbeispiel des bekannten Instruments sind die Scherenarme über einen Gelenkbolzen miteinander verbunden, um den die beiden Scherenarme gegeneinander verschwenkbar sind. Gegenüber der unverbundenen Ausgestaltung der Scherenarme stellt diese Ausgestaltung hinsichtlich der Führung der Scherenarme beim Verschwenken eine Verbesserung dar, jedoch kann auch ein solches Gelenk ein Auseinanderspreizen der Scherenarme quer zur Schneidrichtung nicht vollkommen vermeiden. In der minimal-invasiven Chirurgie besteht nämlich das Bedürfnis nach einer möglichst klein bauenden miniaturisierten Bauweise, so daß die Scherenarme mitunter sehr dünn sind. Da die Scherenarme nur im Bereich des Gelenkbolzens aneinander gehalten werden, kann das Gelenk ein Aufspreizen der Scherenarme quer zur Schneidrichtung im Bereich des distalen Endes der Scherenarme nicht in jedem Fall vermeiden. Wenn der Gelenkbolzen die beiden Scherenarme dicht aneinandergepreßt halten soll, muß der Gelenkbolzen entsprechend mit beiden Scherenarmen fest und spielfrei verbunden werden, was bei einer miniaturisierten Bauweise nicht mit ausreichender dauerhafter Stabilität erreicht werden kann.

Ein weiteres Problem ergibt sich dann, wenn das Instrument, wie es bei dem bekannten Instrument vorgesehen ist, nicht nur zum mechanischen Schneiden, sondern auch zum bipolaren Koagulieren von Gewebe geeignet sein soll.

Um als bipolares Koagulationsinstrument dienen zu können, müssen die beiden Scherenarme als mit Hochfrequenzstrom beaufbeaufschlagbare Elektroden ausgebildet sein, wobei der eine Scherenarm mit dem einem Pol und der andere Scherenarm mit dem anderen Pol der Hochfrequenzspannungsquelle verbunden wird, so daß beide Scherenarme auf unterschiedlichen Potentialen liegen. Dies erfordert es wiederum, die beiden Scherenarme zumindest in ihren sich gegenseitig berührenden Bereichen gegeneinander zu isolieren, damit sich ein Stromfluß bis zu den distalen Enden der Scherenarme ausbilden kann.

Wenn aber wie bei dem bekannten Instrument der Gelenkbolzen auch noch den Anpreßdruck zum Aneinanderpressen der beiden Scherenarme aufbringen muß, muß der Gelenkbolzen mit beiden Scherenarmen verpreßt oder verschraubt sein, so daß der Gelenkbolzen aus Metall hergestellt werden muß. Daraus ergeben sich im Bereich des Gelenkbolzens jedoch Probleme mit der Isolation, so daß im Bereich des Gelenkbolzens ein Kurzschluß oder Kriechströme auftreten können, so daß sich der Stromfluß nicht bis zu den distalen Enden der Scherenarme ausbilden kann. Selbst wenn ein solcher Gelenkbolzen aus Metall mit einer Isolierschicht versehen wird, können Kriechströme nicht vollkommen ausgeschlossen werden, und die Isolierschicht kann sich außerdem im Laufe des Gebrauchs des Instruments abnutzen, so daß eine ausreichende Isolierung der Scherenarme gegeneinander nicht dauerhaft gewährleistet ist.

Ein weiteres chirurgisches Schneidinstrument, mit dem Gewebe auch koaguliert werden kann, ist aus dem Dokument US-A-3 651 811 bekannt. Die Schneidkanten der Scherenarme laufen beim Verschwenken über Kreuz streifend aneinander vorbei. Bei diesem bekannten Instrument sind die beiden Scherenarme über eine Schraube, die gleichzeitig die ortsfeste Drehachse bildet, miteinander verbunden.

Aus dem Dokument US-A-4 452 106 ist eine medizinische Pinzette bekannt, die gelenkig am proximalen Ende des Instruments miteinander verbundene Pinzettenarme aufweist. Die Pinzettenarme sind somit nicht über eine ortsfeste Drehachse miteinander verbunden. Das Instrument weist einen Mechanismus zum Führen der beiden Pinzettenarme in einer einzigen Bewegungsebene bezüglich dem jeweils gegenüberliegenden Pinzettenarm beim Zusammendrücken der Pinzettenarme auf. Hierdurch soll im Wesentlichen ein unerwünschtes seitliches Ausweichen der Pinzettenarme vermieden werden.

Es sind auch teilbare Scheren bekannt, deren Gelenk in der Art einer Schlüssellochverbindung ausgeführt ist. Auch diese Art der Ausführung hat den Nachteil, die Scherenarme nicht ausreichend zu führen und gegen Aufspreizen zu sichern. Auch ist hier eine ausreichende Isolierung der Scherenarme gegeneinander nicht dauerhaft gewährleistet.

Der Erfindung liegt daher die Aufgabe zugrunde, bei einem medizinischen Instrument der eingangs genannten Art eine verbesserte Führung der Scherenarme zu erreichen, wobei das Instrument dennoch leicht reinigbar sein soll.

Erfindungsgemäß wird diese Aufgabe bei einem Instrument der eingangs genannten Art dadurch gelöst, daß zumindest einer der Scherenarme zumindest eine mit diesem verbundene Lasche aufweist, die den anderen Scherenarm außenseitig zumindest teilweise übergreift, und daß die zumindest eine Lasche in einer über die maximal gespreizte Betriebsstellung hinausgehenden Spreizstellung mit dem anderen Schwenkarm außer Eingriff kommt, und daß die Scherenarme in dieser Spreizstellung voneinander trennbar sind.

Die zumindest eine mit dem einen Scherenarm verbundene und den anderen Scherenarm außenseitig zumindest teilweise übergreifende Lasche hat nun die vorteilhafte Wirkung, daß der andere Scherenarm, den die Lasche übergreift, von der Lasche gegen den die Lasche aufweisenden Scherenarm gedrückt wird oder zumindeset an einem Abspreizen gehindert ist, so daß auch beim Schneiden von härterem Gewebe, insbesondere von Gefäßen, gewährleistet ist, daß die Schneidkanten stets streifend aneinander vorbeilaufen. Die Lasche sorgt somit für den erforderlichen Zusammenhalt der beiden Scherenarme, wobei der durch die zumindest eine Lasche erreichbare Anpreßdruck wirkungsvoller ist als der durch einen Gelenkbolzen erzielbare Anpreßdruck, da die Lasche eine größere Anpreßfläche bietet und sich außerdem im Unterschied zu einem Gelenk auch außerhalb einer Schwenkachse positionieren läßt, bspw. nahe am distalen Ende, wo die Gefahr des Abspreizens am größten ist. Durch die erfindungsgemäße Ausgestaltung des Instruments ist nicht nur seine mechanische Schneidwirkung aufgrund einer Führung verbessert, sondern im Fall, daß die Scherenarme zusätzlich über ein Gelenk miteinander verbunden sind, wird dieses Gelenk darüber hinaus entlastet, weil das Gelenk nicht mehr für den Zusammenhalt sorgen muß. Falls ein Gelenk vorgesehen ist, kommt diesem Gelenk somit nur noch die Aufgabe zu, eine Drehachse zu definieren, um die die beiden Scherenarme relativ zueinander verschwenkbar sind. Auch die Eignung des erfindungsgemäßen Instruments als bipolares Koagulationsinstrument in Verbindung mit einer gelenkigen Verbindung der Scherenarme untereinander ist verbessert, weil für das Gelenk ein Gelenkstift verwendet werden kann, der aus einem elektrisch isolierenden Material bestehen kann, bspw. aus einer Keramik, da dieses Gelenk keinen Anpreßdruck aufbringen muß, sondern im wesentlichen kräftefrei ist. Auf diese Weise kann eine elektrische Isolierung der beiden Scherenarme im Bereich eines Gelenks dauerhaft gewährleistet werden.

Die ortsfeste Drehachse hat den Vorteil, daß die Führung der Scherenarme weiter verbessert wird, indem die Scherenarme um eine ortsfeste Drehachse verschwenken. Wie bereits erwähnt, ist dazu jedoch keine Gelenkausgestaltung erforderlich, über die die Scherenarme quer zur Schneidrichtung aneinander gedrückt werden.

Ein weiterer Vorteil, der sich durch die Erfindung eröffnet, besteht darin, die Scherenarme trotz ihrer Führung auf leicht zu handhabende Weise trennbar auszugestalten.

Die Trennbarkeit der Scherenarme voneinander hat den Vorteil einer leichteren Reinigung des Instruments im Bereich der Scherenarme nach dem Gebrauch, insbesondere können sich zwischen den Scherenarmen ansammelnde Gewebereste oder Blut besonders leicht rückstandsfrei entfernt werden. Die Trennbarkeit der beiden Scherenarme ist durch diese Ausgestaltung auch besonders einfach in der Handhabung, da die Scherenarme zum Auseinandernehmen lediglich in die genannte Spreizstellung geöffnet werden müssen, bis die zumindest eine Lasche den anderen Scherenarm freigibt. In Verbindung mit der zuvor genannten Ausgestaltung, daß die beiden Scherenarme um eine Drehachse relativ zueinander verschwenkbar sind, wobei die Drehachse durch einen zumindest von einem der Scherenarme lösbaren Zapfen gebildet wird, ergibt sich ebenfalls eine besonders einfach zu handhabende Trennbarkeit der beiden Scherenarme. Im Betriebszustand sind die Scherenarme durch die zumindest eine Lasche gegen eine unerwünschte Trennung aneinander gesichert, so daß keine weiteren Sicherungsmaßnahmen im Bereich der Drehachse bzw. am Zapfen vorgesehen sein müssen.

Unter einer Lasche im Sinne der vorliegenden Erfindung ist unabhängig von einer Form, Anordnung oder eines Materials ein Abschnitt eines Scherenarms zu verstehen, der von diesem Scherenarm ausgehend den anderen Scherenarm außenseitig zumindest teilweise übergreift, so daß dieser andere Scherenarm zwischen dem einen Scherenarm und diesem Abschnitt zumindest teilweise eingeklammert ist bzw. einklemmbar ist, wobei dies jedoch so zu verstehen ist, daß die Verschwenkbarkeit der Scherenarme weiterhin leichtgängig ist, also keine wesentlich erhöhte Reibung durch die Laschen verursacht wird.

In einer bevorzugten Ausgestaltung weist die zumindest eine Lasche in Richtung quer zur Längsachse der Scherenarme eine solche Ausdehnung auf, daß sie den anderen Scherenarm noch in einer maximal gespreizten Betriebsstellung der Scherenarme zumindest teilweise übergreift.

Wenn die Scherenarme bspw. zum Schneiden von dickerem Gewebe oder dickeren Gefäßen weit auseinander gespreizt werden müssen, ist hierbei von Vorteil, daß eine die Scherenarme zusammenhaltende Führung bereits zu Beginn des auszuführenden Schnitts durch die zumindest eine Lasche bewirkt wird, so daß ein Auseinanderspreizen bzw. Auseinanderbiegen der Scherenarme quer zur Schneidrichtung bereits beim Ansetzen des Schnittes vermieden werden kann.

In einer weiteren bevorzugten Ausgestaltung läuft die zumindest eine Lasche an einem freien Ende keilförmig zu.

Wenn bspw. die Scherenarme eine Spreizstellung einnehmen können, in der die zumindest eine Lasche mit dem anderen Scherenarm außer Eingriff kommt, hat diese Maßnahme den Vorteil, daß das Einfahren des anderen Scherenarms zwischen den einen Scherenarm und die zumindest eine Lasche verbessert wird, und es außerdem ermöglicht wird, die beiden Scherenarme beim Schließen der Scherenarme entlang der Schräge der Lasche mit zunehmendem Anpreßdruck gegeneinander zu drücken.

In einer weiteren bevorzugten Ausgestaltung ist vorgesehen, die Drehachse durch einen zumindest von einem der Scherenarme lösbaren Zapfen zu bilden.

Dieser Zapfen kann demnach lose in jeweils einer Bohrung des einen und des anderen Scherenarms sitzen, ohne mit einem der beiden Scherenarme verpreßt, verschraubt oder auf sonstige Weise fest verbunden werden zu müssen. Bei einer möglichen Ausgestaltung des erfindungsgemäßen Instruments zum zusätzlichen bipolaren Koagulieren hat dies den weiteren Vorteil, daß der Zapfen aus einem Isolatorwerkstoff, bspw. Keramik oder Kunststoff, gefertigt sein kann, da der Zapfen keinen Reibungen oder gar Scher- oder Druckkräften ausgesetzt ist.

In einer weiteren bevorzugten Ausgestaltung ist die zumindest eine Lasche distalseitig der Drehachse angeordnet.

Diese Anordnung der zumindest einen Lasche ist besonders vorteilhaft, da sich der wirksame Schneidbereich der Scherenarme ebenfalls distalseitig der Drehachse befindet und die Neigung, sich quer zur Schneidrichtung aufzuspreizen, dort am größten ist.

Zusätzlich oder alternativ kann jedoch die zumindest eine Lasche proximalseitig der Drehachse angeordnet sein.

Besonders bevorzugt ist es, wenn die zumindest eine Lasche distalseitig und zumindest eine weitere Lasche proximalseitig der Drehachse angeordnet ist.

Bei dieser Ausgestaltung mit zumindest einer Lasche distalseitig und zumindest einer Lasche proximalseitig der Drehachse wird die zuvor genannte gelenkige Verbindung der beiden Scherenarme mittels eines Zapfens vollständig entlastet, weil sowohl auf der distalen Seite der Drehachse an den Scherenarmen angreifende Kräfte als auch proximalseitig der Drehachse an den Scherenarmen angreifende Kräfte, die jeweils ein Aufspreizen der Scherenarme quer zur Schneidrichtung bzw. quer zur Längsrichtung der Scherenarme bewirken können, von den beiden Laschen und somit nicht von dem Gelenk aufgenommen werden.

In einer weiteren bevorzugten Ausgestaltung weisen beide Scherenarme jeweils zumindest eine Lasche auf, und/oder einer der Scherenarme oder beide Scherenarme weist/weisen zumindest zwei axial voneinander beabstandete Laschen auf.

In einer weiteren bevorzugten Ausgestaltung ist die zumindest eine Lasche einstückig mit dem zugehörigen Scherenarm verbunden.

Durch diese Maßnahme wird die Stabilität des Instruments im Bereich der Scherenarme weiter verbessert.

In einer weiteren bevorzugten Ausgestaltung weisen die Scherenarme an ihrem jeweiligen proximalen Ende einen elastischen Biegeabschnitt auf, wobei die Biegeabschnitte durch einen axial verschiebbaren Rohrschaft durch Aufgleiten des Rohrschafts auf die Biegeabschnitte zusammengedrückt und die Scherenarme dadurch aus ihrer Spreizstellung in ihre Schließstellung verschwenkt werden und umgekehrt.

Diese Art der Betätigung der Scherenarme zum Schließen und Öffnen, die an sich aus der US 5,334,198 bekannt ist, führt zu einem konstruktiv einfachen Betätigungsmechanismus für das Instrument.

Bevorzugt ist es dabei jedoch, wenn die Bereiche des einen Scherenarms und Bereiche des anderen Scherenarms, die sich berühren, elektrisch isoliert sind, so daß sich das erfindungsgemäßen Instrument als bipolares Koagulationsinstrument eignet. Die bipolare Koagulation hat den Vorteil, daß sich der Stromfluß auf den Bereich zwischen den auf unterschiedlichen Potentialen liegenden Scherenarmen beschränkt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils beschriebenen Kombination verwendbar sind, ohne allerdings den Rahmen der vorliegenden Erfindung gemäß Anspruch 1 zu verlassen.

Ein Ausführungsbeispiel der Erfindung ist in der Zeichnung dargestellt und wird mit Bezug auf diese nachfolgend Beschreibung näher beschrieben. Es zeigen:
- Fig. 1: ein medizinisches Instrument in einer Gesamtdarstellung in Seitenansicht;
- Fig. 2: eine perspektivische Ansicht von vorn auf den distalen Bereich des Instruments in vergrößertem Maßstab, wobei die Scherenarme des Instruments in ihrer Schließstellung dargestellt sind;
- Fig. 3: eine perspektivische Ansicht von hinten des distalen Bereichs des Instruments in einer gegenüber Fig. 2 um 180° um die Längsachse gedrehten Position, wobei die Scherenarme des Instruments in ihrer maximal gespreizten Betriebsstellung dargestellt sind;
- Fig. 4: eine Draufsicht auf den distalen Bereich des Instruments, teilweise aufgebrochen;
- Fig. 4a): eine Ansicht auf den distalen Bereich von unten in gegenüber Fig. 4 kleinerem Maßstab;
- Fig. 5: einen Ausschnitt aus Fig. 4 in noch weiter vergrößertem Maßstab im Längsschnitt;
- Fig. 6: den ersten Scherenarm des Instruments in Alleinstellung;
- Fig. 7: den zweiten Scherenarm des Instruments in Alleinstellung; und
- Fig. 8a) bis c): die Funktionsweise des Trennens bzw. Zusammensetzens der beiden Scherenarme.

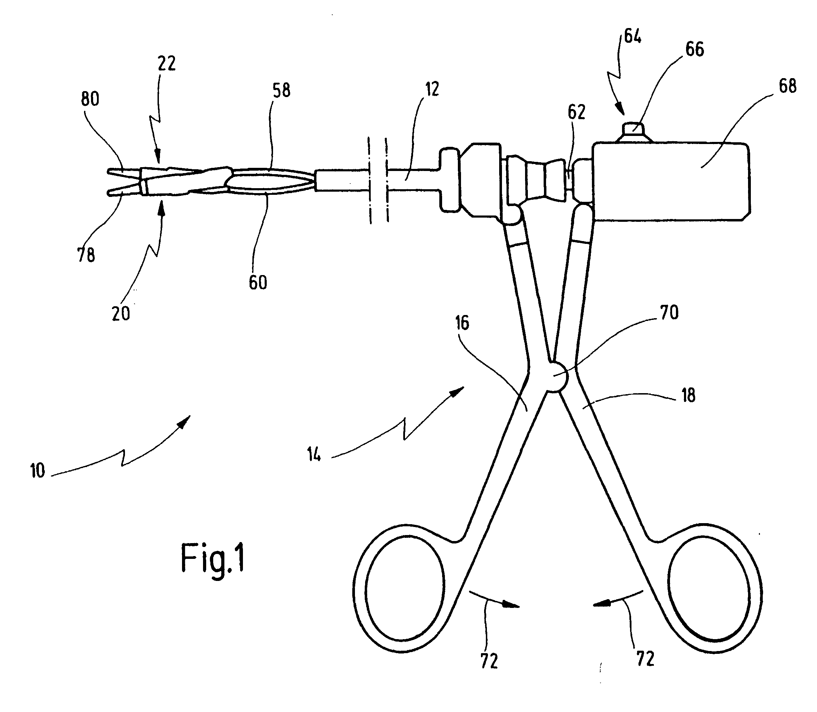

In Fig. 1 ist ein mit dem allgemeinen Bezugszeichen 10 versehenes medizinisches Instrument zum Schneiden von Gewebe im menschlichen oder tierischen Körper dargestellt, das in der minimal-invasiven Chirurgie verwendet wird.

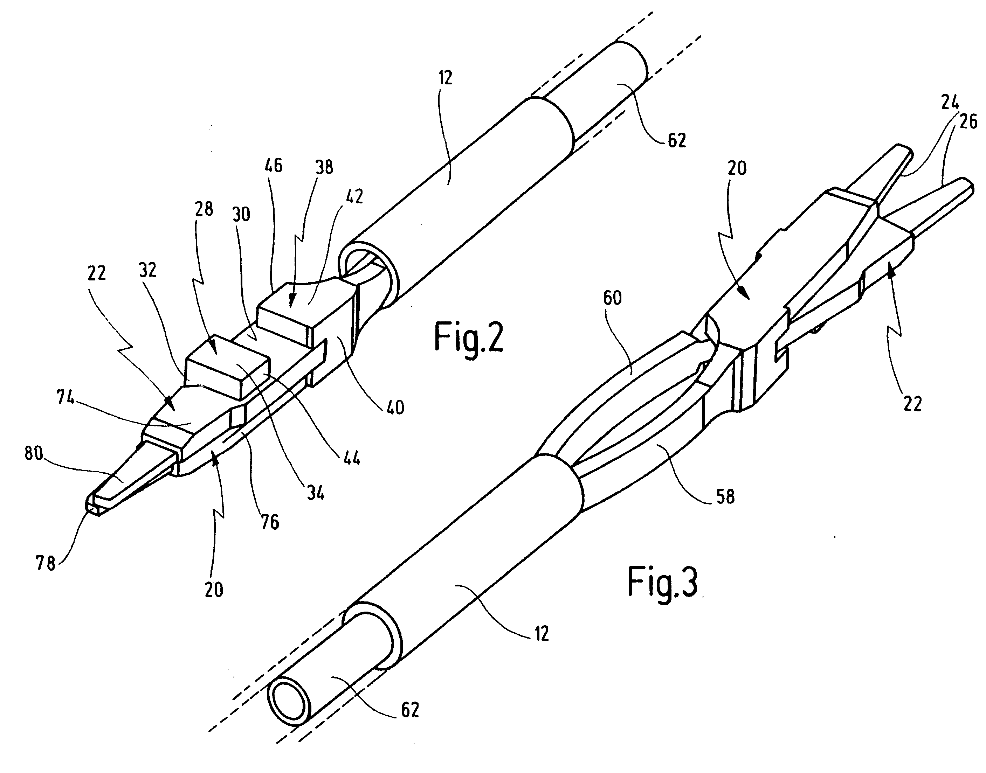

Einzelheiten des Instruments 10 sind in den Fig. 2 bis 7 dargestellt.

Mit dem Instrument 10 kann beliebiges hartes oder weiches Gewebe sowohl mechanisch geschnitten als auch bipolar koaguliert werden.

Das Instrument 10 weist gemäß Fig. 1 allgemein einen Rohrschaft 12, eine Handhabe 14 mit einem ersten Griffteil 16 und einem zweiten Griffteil 18 sowie einen ersten Scherenarm 20 und einen zweiten Scherenarm 22 auf, wobei der erste Scherenarm 20 und der zweiten Scherenarm 22 gegeneinander verschwenkbar sind. Der Rohrschaft 12 ist zwischen den distal angeordneten Scherenarmen 20 und 22 und der proximal angeordneten Handhabe 14 angeordnet. Das Zusammenwirken der Handhabe 14 mit dem Rohrschaft 12 und den Scherenarmen 20 und 22 wird später noch näher beschrieben.

Mit Bezug auf Fig. 2 bis 7 wird zunächst die Ausgestaltung der Scherenarme 20 und 22 näher beschrieben.

Der erste Scherenarm 20 und der zweite Scherenarm 22 sind in ihrer Längsrichtung gerade ausgebildet, wobei die Scherenarme 20 und 22 jedoch auch in Längsrichtung gekrümmt bzw. gekröpft ausgebildet sein können. Eine gekröpfte Ausbildung kann die mechanische Schneidwirkung des Instruments 10 noch weiter verbessern.

Der erste Scherenarm 20 weist eine erste Schneidkante 24 auf, während der zweite Scherenarm 22 eine zweite Schneidkante 26 aufweist. Die Schneidkanten 24 und 26 laufen beim Verschwenken der Scherenarme 20 und 22 über Kreuz streifend aneinander vorbei. Während die Scherenarme 20 und 22 in Fig. 3 in ihrer maximal gespreizten Betriebsstellung dargestellt sind, von der ausgehend im Betrieb des Instruments 10 ein Schnitt angesetzt wird, sind die Scherenarme 20 und 22 in Fig. 2 in ihrer Schließstellung dargestellt, nachdem also ein Schnitt ausgeführt wurde.

Wie später noch näher beschrieben wird, sind die Scherenarme 20 und 22 in ihre in Fig. 3 dargestellte Offenstellung federelastisch vorgespannt, d.h. die Scherenarme 20 und 22 nehmen die in Fig. 3 dargestellte maximal gespreizte Betriebsstellung selbsttätig ein.

Der erste Scherenarm 20 weist eine erste Lasche 28 auf, wobei die Lasche 28 mit dem ersten Scherenarm 20 verbunden ist, und zwar im vorliegenden Beispiel einstückig.

Die erste Lasche 28 übergreift den zweiten Scherenarm 22, wie aus Fig. 2 hervorgeht, außenseitig zumindest teilweise. Außenseitig bedeutet, daß die erste Lasche 28 den zweiten Scherenarm 22 auf dessen dem ersten Scherenarm 20 abgewandten Außenseite 30 übergreift und somit die Scherenarme 20 und 22 zusammenhält.

Die erste Lasche 28 weist etwa die Form eines L auf, wobei ein erster Schenkel 32 der ersten Lasche 28 etwa senkrecht zur Außenseite 30 und damit senkrecht zur Hauptebene der Scherenarme 20 und 22 und ein zweiter Schenkel 34 parallel zur Außenseite 30 verläuft. Der erste Schenkel 32 und der zweite Schenkel 34 verlaufen etwa senkrecht zueinander und weisen jeweils die Form eines Rechtecks auf.

Die erste Lasche 28 weist in Richtung quer zur Längsachse der Scherenarme 20 und 22 eine solche Ausdehnung auf, daß sie den zweiten Scherenarm 22 bis in die maximal gespreizte Betriebsstellung der Scherenarme 20 und 22, wie in Fig. 3 dargestellt, zumindest teilweise noch übergreift.

Der zweite Scherenarm 22 wird demnach durch die erste Lasche 28 gegen den ersten Scherenarm 20 gedrückt, so daß ein Aufspreizen der Scherenarme 20 und 22 in Richtung quer zur Längsrichtung bzw. quer zur Schneidrichtung vermieden wird, und zwar über den gesamten Verschwenkbereich der Scherenarme 20 und 22 zwischen der maximal gespreizten Betriebsstellung in Fig. 3 und der Schließstellung in Fig. 2.

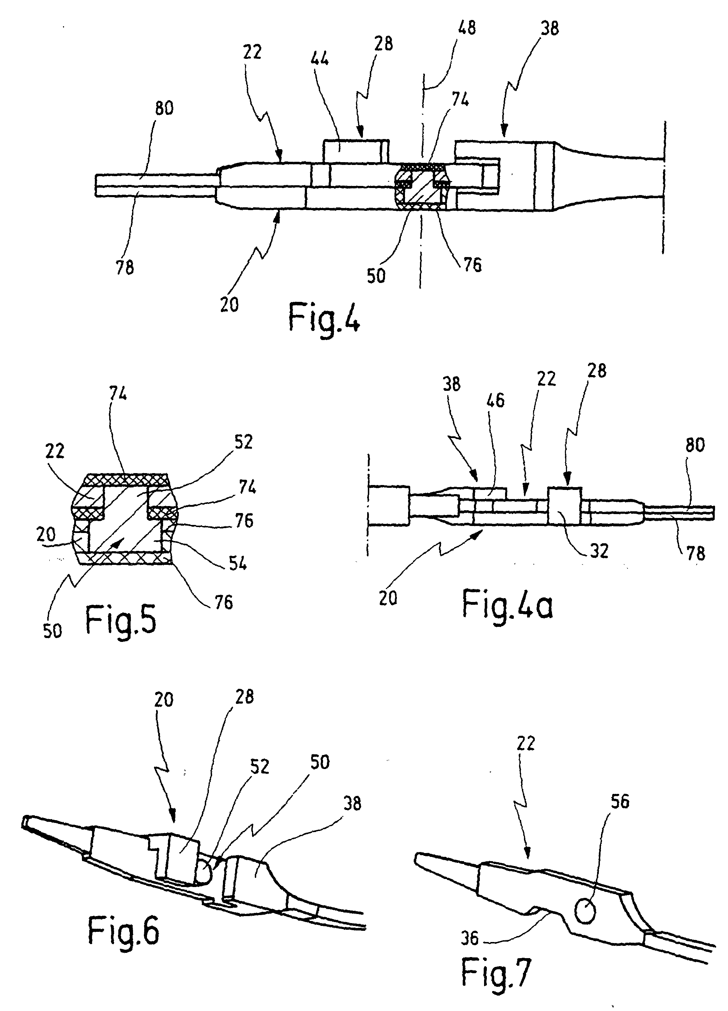

Wie insbesondere aus Fig. 7 hervorgeht, ist der zweite Scherenarm 22 im Bereich des ersten Schenkels 32 der ersten Lasche 28 mit einer Ausnehmung 36 versehen, in der der erste Schenkel 32 in der in Fig. 2 dargestellten Schließstellung der Scherenarme 20 und 22 zu liegen kommt, wodurch eine unerwünschte Verbreiterung der Scherenarme 20 und 22 durch das Vorsehen der erste Lasche 28 vermieden wird.

Der erste Scherenarm 20 weist weiterhin eine zweite Lasche 38 auf, die wiederum mit dem ersten Scherenarm 20 verbunden ist, und die den zweiten Scherenarm 22 ebenfalls außenseitig zumindest teilweise hintergreift. Die zweite Lasche 38 ist ebenfalls etwa L-förmig ausgebildet, und weist demnach einen ersten Schenkel 40 und einen zweiten Schenkel 42 auf, die dem ersten Schenkel 32 bzw. dem zweiten Schenkel 34 der ersten Lasche 28 entsprechen. Die Formgebung des ersten Schenkels 40 und des zweiten Schenkels 42 der zweiten Lasche 38 unterscheidet sich zwar von der Formgebung des ersten Schenkels 32 und des zweiten Schenkels 34 der ersten Lasche 28, jedoch ist die Formgebung der Laschen 28 und 38 nicht kritisch.

Die erste Lasche 28 und die zweite Lasche 38 übergreifen den zweiten Scherenarm 22 von jeweils gegenüberliegenden Seiten her, d.h. ein freies Ende 44 der ersten Lasche 28 und ein freies Ende 46 der zweiten Lasche 38 liegen sich bezüglich der Längsachse der Scherenarme 20 und 22 gegenüber, wie sich aus Fig. 4 und Fig. 4a) ergibt.

Gemäß Fig. 4 sind der erste Scherenarm 20 und der zweite Scherenarm 22 des weiteren um eine ortsfeste Drehachse 48 gegeneinander verschwenkbar. Wie sich aus Fig. 4 ergibt, ist dabei die erste Lasche 28 distalseitig der Drehachse 48 und die zweite Lache 38 proximalseitig der Drehachse 48 angeordnet. Von der zweiten Lasche 38 werden die Scherenarme 20 und 22 proximalseitig der Drehachse 48 zusammengehalten, während die erste Lasche 28 dafür sorgt, daß die Scherenarme 20 und 22 distalseitig der Drehachse 48 zusammengehalten werden.

Die Drehachse 48 wird durch einen Zapfen 50 gebildet. Der Zapfen 50 ist insgesamt zylindersymmetrisch ausgebildet. Der Zapfen 50 weist einen Abschnitt 52 kleineren Durchmessers und einen Abschnitt 54 größeren Durchmessers auf.

Der Abschnitt 54 größeren Durchmessers ist in einer Bohrung in dem ersten Scherenarm 20 eingesetzt, während der Abschnitt 52 kleineren Durchmessers in eine entsprechende Bohrung 56 des zweiten Scherenarms 22 eingesetzt ist. Der Zapfen 50 ist sowohl in dem ersten Scherenarm 20 als auch in dem zweiten Scherenarm 22 lose aufgenommen, d.h. der Zapfen 50 kann sowohl von dem ersten Scherenarm 20 als auch von dem zweiten Scherenarm 22 gelöst werden. Es kann aber auch vorgesehen sein, den Zapfen 50 in dem ersten Scherenarm 20 unverlierbar einzubetten, und lediglich in den zweiten Scherenarm 22 lose einzusetzen.

Um den Zapfen 50 können die Scherenarme 20 und 22 im Gebrauch des Instruments 10 gegeneinander verschwenken. Die lose Verbindung des Zapfens 50 mit den Scherenarmen 20 und 22 wird durch das Vorsehen zumindest der ersten Lasche 28 ermöglicht, im vorliegenden Fall durch die erste Lasche 28 und die zweite Lasche 38, die nämlich die Scherenarme 20 und 22 im Betrieb des Instruments 10 untrennbar zusammenzuhalten und aneinanderdrücken. Der Zapfen 50 hat demgegenüber lediglich die Funktion, die ortsfeste Drehachse 48 zu definieren. In der getrennten Darstellung des ersten Scherenarmes 20 in Fig. 6 ist der Abschnitt 52 kleineren Durchmessers des Zapfens 50 zu sehen, während die Bohrung 56, in die der Abschnitt 52 des Zapfens 50 im zusammengesetzten Zustand eingesetzt ist, in Fig. 7 zu sehen ist.

An dem Zapfen 50 liegen die Scherenarme 20 und 22 über Kreuz.

Im zusammengesetzten Zustand der Scherenarme 20 und 22 ist der Zapfen 50 von außen nicht zu sehen.

Der erste Scherenarm 20 und der zweite Scherenarm 22 weisen an ihrem jeweiligen proximalen Ende jeweils einen elastischen Biegeabschnitt 58 und 60 auf. Die Biegeabschnitte 58 und 60 sind in der Art von Blattfedern ausgebildet, die in ihrer Ruhestellung eine nach außen gekrümmte Stellung einnehmen. Über die Biegeabschnitte 58 und 60 sind die Scherenarme 20 und 22 mit einem Innenrohr 62 verbunden, das wiederum über einen Rastmechanismus 64 mit einem Rastknopf 66 mit einem Gehäuse 68 am proximalen Ende des Instruments 10 lösbar verrastet ist. Das Gehäuse 68 ist gleichzeitig als Steckergehäuse zum Anschließen eines mit einer Hochfrequenzspannungsquelle verbundenen Stromkabels ausgebildet.

Zunächst wird jedoch die Funktionsweise des Instruments 10 zum mechanischen Schneiden von Gewebe beschrieben.

Das erste Griffteil 16 und das zweite Griffteil 18 der Handhabe 14 sind über ein Gelenk 70 relativ zueinander beweglich miteinander verbunden. Das Griffteil 16 ist fest mit dem Rohrschaft 12 verbunden. Der Rohrschaft 12 seinerseits ist relativ zu dem Innenrohr 62 und somit zu den Biegeabschnitten 58 und 60 der Scherenarme 20 und 22 axial verschiebbar.

Durch Zusammendrücken der Griffteile 16 und 18 in Richtung von Pfeilen 72 wird der Rohrschaft 12 ausgehend von der in Fig. 1 bzw. Fig. 3 dargestellten Betriebsstellung, die die maximal gespreizte Betriebsstellung der Scherenarme 20 und 22 darstellt, axial nach distal verschoben, gleitet dabei auf die Biegeabschnitte 58 und 60 der Scherenarme 20 und 22 auf, wodurch diese in die in Fig. 2 dargestellte Schließstellung bewegt werden, wodurch die Schneidkanten 24 und 26 über Kreuz streifend aneinander vorbeilaufen. Die Laschen 28 und 38 sorgen dabei beim Schneiden von Gewebe bzw. Gefäßen für den erforderlichen Anpreßdruck der Scherenarme 20 und 22 und damit der Schneidkanten 24 und 26 aneinander. Der die Drehachse 48 bildende Zapfen 50 ist dabei im wesentlichen kräftefrei und sorgt lediglich dafür, daß die Drehachse 48 definiert ist.

Wie bereits erwähnt, ist das Instrument 10 nicht nur zum mechanischen Schneiden, sondern auch zum bipolaren Koagulieren von Gewebe geeignet.

Dazu sind die Scherenarme 20 und 22 als mit Hochfrequenzstrom beaufschlagbare Elektroden ausgebildet, wobei der erste Scherenarm 20 und der zweite Scherenarm 22 elektrisch isoliert voneinander mit dem einen bzw. anderen Pol der Hochfrequenzspannungsquelle verbindbar ist.

Die Scherenarme 20 und 22 einschließlich der Laschen 28 und 38 sind an sich metallisch ausgebildet, wobei die Scherenarme 20 und 22 in den sich gegenseitig berührenden Bereichen jeweils mit einer Isolierung 74 bzw. 76 allseitig umgeben sind. Auch die Laschen 28 und 138 sind elektrisch isoliert. Lediglich distale Enden 78 und 80 der Scherenarme 20 und 22 sind metallisch blank. Die Stromzuführung zu den distalen Enden 78 und 80 der Scherenarme 20 und 22 erfolgt ausgehend von entsprechenden nicht dargestellten Kontakten in dem Gehäuse 68 über Zuleitungen (nicht dargestellt) in dem Innenrohr 62 durch die ebenfalls außenseitig isolierten Biegeabschnitte 58 und 60 zu den distalen Enden 78 und 80 der Scherenarme 20 und 22.

Der Zapfen 50 ist dabei aus einem Isolatorwerkstoff gebildet, bspw. einer Keramik oder einem Kunststoff, kann jedoch aus einem Metall mit einer isolierenden Beschichtung gebildet sein.

In der in Fig. 1 und 3 gezeigten maximal gespreizten Betriebsstellung, in der sich die distalen Enden 78 und 80 der Scherenarme 20 und 22 nicht berühren, kann durch Einschalten des Hochfrequenzstroms Gewebe vor dem Schneiden oder nach dem Schneiden bipolar koaguliert werden, wobei sich der Stromfluß auf den Bereich zwischen den distalen Enden 78 und 80 der Scherenarme 20 und 22 konzentriert. Ein Kurzschluß oder ein Kriechstrom im Bereich des Zapfens 50 tritt dabei nicht auf, da der Zapfen 50 aus einem Isolatorwerkstoff gebildet ist, was wiederum dadurch möglich ist, daß der Zapfen 50 nicht den Anpreßdruck der Scherenarme 20 und 22 aneinander aufbringen muß.

Während vorstehend ein Ausführungsbeispiel für die Ausgestaltung und Anordnung der ersten Lasche 28 und der zweiten Lasche 38 beschrieben wurde, so versteht es sich, daß die Anordnung und Ausgestaltung der Laschen 28 und 38 nicht darauf beschränkt ist. Es kann bspw. auch vorgesehen sein, nur eine Lasche oder mehr als zwei Laschen vorzusehen, oder bspw. beide Scherenarme mit zumindest einer Lasche auszugestalten.

Des weiteren kann auch das freie Ende 44 bzw. 46 der Laschen 28 und 38 keilförmig angeschrägt sein.

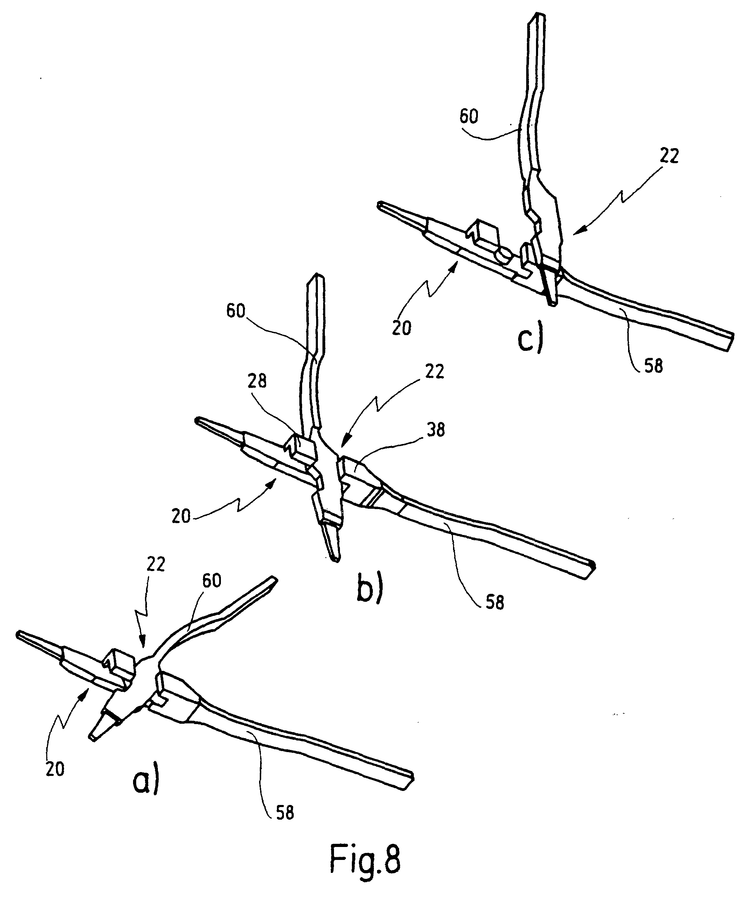

Gemäß einer weiteren Eigenschaft des Instruments 10 sind der erste Scherenarm 20 und der zweite Scherenarm 22 voneinander trennbar, wie nachfolgend mit Bezug auf Fig. 8a) bis c) beschrieben wird.

Nachdem zunächst die Baueinheit aus dem Innenrohr 62, den Biegeabschnitten 58, 60 und den Scherenarmen 20 und 22 durch Auslösen des Rastmechanismus 64 von dem Gehäuse 68 und somit von der Handhabe 14 abgenommen wurden, werden die Biegeabschnitte 58 und 60 von dem Innenrohr 62 gelöst.

Der zweite Scherenarm 22 läßt sich dann relativ zu dem ersten Scherenarm 20 über die maximal gespreizte Betriebsstellung gemäß Fig. 3 hinaus um die durch den Zapfen 50 gebildete Drehachse 48 verschwenken, wie in Fig. 8a) dargestellt ist.

Der zweite Scherenarm 22 wird, wie in Fig. 8b) darstellt ist, dabei soweit relativ zu dem ersten Scherenarm 20 verschwenkt, daß die erste Lasche 28 und die zweite Lasche 38 mit dem zweiten Scherenarm 22 außer Eingriff kommen, wozu die erste Lasche 28 und die zweite Lasche 38 entsprechend der Breite des zweiten Scherenarms 22 axial voneinander beabstandet sind. In dieser Stellung stehen der erste Scherenarm 20 und der zweite Scherenarm 22 etwa senkrecht zueinander.

In dieser Stellung läßt sich dann der zweite Scherenarm 22 von dem ersten Scherenarm 20 abheben, da der Zapfen 50 lose in dem zweiten Scherenarm 22 eingesetzt ist. In Fig. 8c) sind der erste Scherenarm 20 und der zweite Scherenarm 22 getrennt voneinander dargestellt.

Zum Zusammensetzen des Instruments 10 wird dann entsprechend in umgekehrter Reihenfolge vorgegangen.

## Patentansprüche

1. Medizinisches Instrument zum Schneiden und bipolaren Koagulieren von Gewebe im menschlichen oder tierischen Körper, mit zwei um eine ortsfeste Drehachse (48) relativ zueinander verschwenkbaren Scherenarmen (20, 22), an denen die Drehachse (48) angeordnet ist, die jeweils eine Schneidkante (24, 26) aufweisen; wobei die Schneidkanten (24, 26) beim Verschwenken der Scherenarme (20, 22) überkreuz streifend aneinander vorbeilaufen, und wobei ferner die Scherenarme (20, 22) als mit Hochfrequenzstrom beaufschlagbare Elektroden ausgebildet sind, wobei sich gegenseitig berührende Bereiche des einen Scherenarms (20) und des anderen Scherenarms (22) elektrisch isoliert sind, **dadurch gekennzeichnet, daß** zumindest einer der Scherenarme (20) zumindest eine mit diesem verbundene Lasche (28, 38) aufweist, die den anderen Scherenarm (22) außenseitig zumindest teilweise übergreift, und daß die zumindest eine Lasche (28, 38) in einer über die maximal gespreizte Betriebsstellung hinausgehenden Spreizstellung mit dem anderen Scherenarm (22) außer Eingriff kommt, und daß die Scherenarme (20, 22) in dieser Spreizstellung voneinander trennbar sind.

2. Instrument nach Anspruch 1, **dadurch gekennzeichnet, daß** die zumindest eine Lasche (28, 38) in Richtung quer zur Längsachse der Scherenarme (20, 22) eine solche Ausdehnung aufweist, daß sie den anderen Scherenarm (22) noch in einer maximal gespreizten Betriebsstellung der Scherenarme (20, 22) zumindest teilweise übergreift.

3. Instrument nach Anspruch 1 oder 2, **dadurch gekennzeichnet, daß** die zumindest eine Lasche (28, 38) an einem freien Ende (44, 46) keilförmig zuläuft.

4. Instrument nach einem der Ansprüche 1 bis 3, **dadurch gekennzeichnet, daß** die Drehachse (48) durch einen zumindest von einem der Scherenarme (22) lösbaren Zapfen (50) gebildet wird.

5. Instrument nach einem der Ansprüche 1 bis 4, **dadurch gekennzeichnet, daß** die zumindest eine Lasche (28) distalseitig der Drehachse (48) angeordnet ist.

6. Instrument nach einem der Ansprüche 1 bis 5, **dadurch gekennzeichnet, daß** die zumindest eine Lasche (38) proximalseitig der Drehachse (48) angeordnet ist.

7. Instrument nach einem der Ansprüche 1 bis 6, **dadurch gekennzeichnet, daß** die zumindest eine Lasche (28) distalseitig und zumindest eine weitere Lasche (38) proximalseitig der Drehachse (48) angeordnet ist.

8. Instrument nach einem der Ansprüche 1 bis 7, **dadurch gekennzeichnet, daß** beide Scherenarme (20, 22) jeweils zumindest eine Lasche (28, 38) aufweisen.

9. Instrument nach einem der Ansprüche 1 bis 8, **dadurch gekennzeichnet, daß** einer der Scherenarme (20) oder beide Scherenarme (20, 22) zumindest zwei axial voneinander beabstandete Laschen (28, 38) aufweist/aufweisen.

10. Instrument nach einem der Ansprüche 1 bis 9, **dadurch gekennzeichnet, daß** die zumindest eine Lasche (28, 38) einstückig mit dem zugehörigen Scherenarm (20) verbunden ist.

11. Instrument nach einem der Ansprüche 1 bis 10, **dadurch gekennzeichnet, daß** die Scherenarme (20, 22) an ihrem jeweiligen proximalen Ende einen elastischen Biegeabschnitt (58, 60) aufweisen, wobei die Biegeabschnitte (58, 60) durch einen axial verschiebbaren Rohrschaft (12) durch Aufgleiten des Rohrschafts (12) auf die Biegeabschnitte (58, 60) zusammengedrückt und die Scherenarme (20, 22) **dadurch** aus ihrer Spreizstellung in ihre Schließstellung verschwenkt werden.

## Claims

1. A medical instrument for cutting and bipolar coagulating of tissue in the human or animal body, comprising two scissor arms (20, 22) pivotal with respect to one another about a pivot axis (48) fixed in space and arranged at the scissor arms (20, 22), each scissor arm (20, 22) having a cutting edge (24, 26), wherein the cutting edges (24, 26) slide across one another when pivoting the scissor arms (20, 22), and wherein the scissor arms (20, 22) are configured as electrodes to be supplied with high frequency current, wherein regions of the one scissor arm (20) and the other scissor arm (22), which mutually are in contact, are electrically insulated, **characterised in that** at least one of the scissor arms (20) comprises at least one bracket (28, 38) connected thereto, which at least partially extends over the outer side of the other scissor arm (22), and that the at least one bracket (28, 38) disengages with the other scissor arm (22) in a spread position going beyond the maximally spread operational position, and that the scissor arms (20, 22) are separable from one another in this spread position.

2. The instrument of claim 1, **characterized in that** the at least one bracket (28, 38) has an extension in transverse direction to the longitudinal axis of the scissor arms (20, 22), such that it at least partially extends over the other scissor arm (22) in a maximally spread operational position of the scissor arms (20, 22).

3. The instrument of claim 1 or 2, **characterized in that** the at least one bracket (28, 38) is wedge-shaped at a free end (44, 46).

4. The instrument of anyone of claims 1 through 3, **characterized in that** the pivot axis (48) is formed by a pin (50) releasable from at least one of the scissor arms (22).

5. The instrument of anyone of claims 1 through 4, **characterized in that** the at least one bracket (28) is arranged at the distal side of the pivot axis (48).

6. The instrument of anyone of claims 1 through 5, **characterized in that** the at least one bracket (38) is arranged at the proximal side of the pivot axis (48).

7. The instrument of anyone of claims 1 through 6, **characterized in that** the at least one bracket (28) is arranged at the distal side and at least one further bracket (38) is arranged at the proximal side of the pivot axis (48).

8. The instrument of anyone of claims 1 through 7, **characterized in that** the two scissor arms (20, 22) each comprise at least one bracket (28, 38).

9. The instrument of anyone of claims 1 through 8, **characterized in that** one of the scissor arms (20) or both of the scissor arms (20, 22) comprise(s) at least two brackets (28, 38) spaced axially from one another.

10. The instrument of anyone of claims 1 through 9, **characterized in that** the at least one bracket (28, 38) is integrally connected with the associated scissor arm (20).

11. The instrument of anyone of claims 1 through 10, **characterized in that** the scissor arms (20, 22) comprise an elastic bending portion (58, 60) at their respective proximal ends, wherein the bending portions (58, 60) are compressed by an axially shiftable tubular shaft when sliding the tubular shaft (12) over the bending portions (58, 60), whereby the scissor arms (20, 22) are pivoted to their closed position from their spread position.

## Revendications

1. Instrument médical pour couper et coaguler de manière bipolaire des tissus de corps humains ou animaux, comportant deux bras de ciseaux (20, 22) pouvant pivoter l'un par rapport à l'autre autour d'un axe de rotation (48) fixe et sur lesquels est disposé l'axe de rotation (48), lesquels bras de ciseaux présentent chacun une arête de coupe (24, 26), les arêtes de coupe (24, 26) passant l'une devant l'autre en se croisant et se frottant, lors du pivotement des bras de ciseaux (20, 22), et les bras de ciseaux (20, 22) étant réalisés en outre comme électrodes pouvant être soumises à un courant à haute fréquence, les zones en contact réciproque d'un bras de ciseaux (20) et de l'autre bras de ciseaux (22) étant isolées électriquement, **caractérisé en ce qu'**au moins l'un des bras de ciseaux (20) comporte au moins une éclisse (28, 38) reliée à celui-ci, laquelle passe au moins en partie extérieurement sur l'autre bras de ciseaux (22), et **en ce que** la au moins une éclisse (28, 38) se dégage de l'autre bras de ciseaux (22) dans une position d'écartement allant au-delà de la position de fonctionnement écartée au maximum, et **en ce que** les bras de ciseaux (20, 22) peuvent être séparés l'un de l'autre dans cette position d'écartement.

2. Instrument selon la revendication 1, **caractérisé en ce que** la au moins une éclisse (28, 38) présente, dans une direction transversale à l'axe longitudinal des bras de ciseaux (20, 22), une étendue telle qu'elle passe encore au moins en partie sur l'autre bras de ciseaux (22) dans une position de fonctionnement écartée au maximum des bras de ciseaux (20, 22).

3. Instrument selon la revendication 1 ou 2, **caractérisé en ce que** la au moins une éclisse (28, 38) se termine en forme de coin à une extrémité libre (44, 46).

4. Instrument selon l'une des revendications 1 à 3, **caractérisé en ce que** l'axe de rotation (48) est formé par une tige (50) pouvant être séparée au moins de l'un des bras de ciseaux (22).

5. Instrument selon l'une des revendications 1 à 4, **caractérisé en ce que** la au moins une éclisse (28) est disposée sur le côté distal de l'axe de rotation (48).

6. Instrument selon l'une des revendications 1 à 5, **caractérisé en ce que** la au moins une éclisse (38) est disposée sur le côté proximal de l'axe de rotation (48).

7. Instrument selon l'une des revendications 1 à 6, **caractérisé en ce que** la au moins une éclisse (28) est disposée sur le côté distal et au moins une autre éclisse (38) est disposée sur le côté proximal de l'axe de rotation (48).

8. Instrument selon l'une des revendications 1 à 7, **caractérisé en ce que** les deux bras de ciseaux (20, 22) comportent chacun au moins une éclisse (28, 38).

9. Instrument selon l'une des revendications 1 à 8, **caractérisé en ce qu'**au moins l'un des bras de ciseaux (20) ou les deux bras de ciseaux (20, 22) comporte(nt) au moins deux éclisses (28, 38) espacées axialement l'une de l'autre.

10. Instrument selon l'une des revendications 1 à 9, **caractérisé en ce que** la au moins une éclisse (28, 38) est reliée d'une seule pièce au bras de ciseaux (20) correspondant.

11. Instrument selon l'une des revendications 1 à 10, **caractérisé en ce que** les bras de ciseaux (20, 22) comportent un tronçon de pliage (58, 60) élastique à leur extrémité proximale respective, les tronçons de pliage (58, 60) étant comprimés par une tige tubulaire (12) pouvant coulisser axialement, par glissement de la tige tubulaire (12) sur les tronçons de pliage (58, 60), et les bras de ciseaux (20, 22) sont de ce fait pivotés de leur position d'écartement à leur position de fermeture.
